# EUROPEAN PATENT SPECIFICATION

(11) **EP 3 747 326 B1**
(45) Date of publication and mention of the grant of the patent: **06.09.2023**
(21) Application number: 20169123.5
(22) Date of filing: 09.04.2020
(51) Int. Cl.: A47K 5/12, A61B 90/80, A61G 7/00, A45F 5/02

(54) **WEARABLE DISPENSER AND ARTICLE OF CLOTHING AND METHOD FOR WASHING A NUMBER OF CARE RECIPIENTS**
TRAGBARER SPENDER UND KLEIDUNGSSTÜCK UND VERFAHREN ZUM WASCHEN EINER ANZAHL VON PFLEGEBEDÜRFTIGEN PERSONEN
DISTRIBUTEUR PORTABLE ET ARTICLE DE VÊTEMENT ET PROCÉDÉ POUR LAVER UN CERTAIN NOMBRE DE PATIENTS

(30) Priority: 09.04.2019 NL 2022907
(43) Date of publication of application: 09.12.2020
(73) Proprietor: JOSH IP II B.V., 9521 JA Nieuw-Buinen (NL)
(72) Inventor: HUIZINGA, Jozef, 9521 JA Nieuw-Buinen (NL)
(74) Representative: V.O.

(56) References cited:
- US-A1- 2004 068 824
- US-A1- 2011 067 728
- US-A1- 2013 099 929
- US-B2- 7 988 020

## Description

The invention relates to a wearable dispenser, an article of clothing provided with the wearable dispenser, and a method for washing a number of care recipients.

### BACKGROUND

It is known to wash care recipients in, for example, hospitals and care institutions with the aid of prepacked, moist wipes/washcloths. These moist wipes/washcloths are purchased ready-made and have been premoistened, and hermetically and antiseptically packed. Immediately prior to use, the wipes/washcloths while in the package are warmed, for example in a microwave or an autoclave. Warming is done to avoid the uncomfortable feel of a wet, cold washcloth and thus to increase patient comfort during the treatment.

A great disadvantage of this method resides in particular in the time it takes to warm up the moist wipes/washcloths. This is because taking care of the care recipient takes place at the care recipient's bed, whereas warming of the wipes/washcloths takes place in a central appliance. This means that a care provider, after taking care of a first care recipient, must first walk to the central appliance and there proceed to warm a package for a next care recipient. In this way, the care provider is constantly walking to and back from the central appliance.

Another disadvantage of this method is the price of the moist ready-made wipes/washcloths. One of the causes of the steep price resides in the necessity to prevent the presence of germs (pathogens), such as fungi and bacteria, in the cloths and/or the liquid during production.

Indeed, such fungi and bacteria can multiply rapidly within the gastight package. Upon a package being opened by a caregiver, the presence of, for example, bacteria need not even be manifest directly, so that the cloths will still be used for washing a patient or caregiver, which entails a health hazard. The production process must therefore take place under sterile conditions. Moreover, of each batch of cloths produced, a few samples have to be tested in a laboratory for the presence of germs. Only after such laboratory tests can a production batch be cleared for sale. It will be clear that producing in a sterile environment and having to subject samples from every production batch to laboratory tests is costly.

Moreover, the storage life of the packed moist wipe cloths is limited, which makes stock control more difficult. One of the causes of this limited storage life is that even in the closed package, in time, a degree of drying out of the moist cloths will occur, which is generally unwanted and can therefore be tolerated to a limited extent only.

Yet another disadvantage is that multiple moistened cloths have been placed in a package which is warmed prior to use. After opening of the package, the moistened cloths have only a very limited storage life, because bacteria and/or fungi can rapidly reproduce on the warmed, moist cloth. This has as a consequence that, in practice, when the washing of a care recipient has been finished, unused moist cloths are often thrown away, which, besides being a waste of money, also adversely affects the environment.

### SUMMARY

The object of the invention is to provide a solution which reduces the disadvantages of the current method at least partly, and preserves the advantages. The invention provides to this end a wearable dispenser according to claim 1. More particularly, the invention provides a wearable dispenser comprising a carrying support, a wash lotion holder, a dispenser head and a transport mechanism. The wash lotion holder is attached to the carrying support and is configured for holding a wash lotion. The dispenser head is attached to the carrying support and is provided with at least one opening which is configured for dispensing a dose of the wash lotion to a caregiver. The transport mechanism is configured for transporting the dose of wash lotion from the wash lotion holder to the dispenser head in order to allow dispensing of the wash lotion to the caregiver. The at least one opening of the dispenser head comprises multiple openings through which the dose of wash lotion, in operation, is dispensed. The dispenser head is provided with a front cap which is movable in a direction substantially perpendicular to a front surface of the front cap for the purpose of providing an activation act for activating the transport mechanism for dispensing the dose of wash lotion via the openings. The multiple openings are provided in the front surface of the front cap, such that pressing in with a washcloth on the front cap leads directly to moistening of the washcloth.

The caregiver of the wearable dispenser, who will typically be a care provider in, for example, a hospital or a care institution, can walk with the wearable dispenser from care recipient to care recipient. For example, the caregiver will hold a dry washcloth against the dispenser head, so that the dispensed dose of wash lotion is adsorbed into the washcloth. Thus, the washcloth is moistened on the spot and the caregiver does not have to walk to a central space for a moist washcloth. This will considerably reduce the time that is needed for washing multiple care recipients.

It is not necessary here to warm the washcloth prior to use. The cause of the uncomfortable feel of a wet, cold washcloth is not mainly the temperature of the moist washcloth, but the heat which the moistened washcloth draws from the skin upon contacting it. As the dosage can be precisely tuned to the situation, the dose of wash lotion can be kept as small as possible. In consequence of this, the heat being thereby drawn from the skin by the moistened washcloth is practically imperceptible and is negligible.

Due to the wearable dispenser moistening the washcloths at the location of the care recipient, it is not necessary to use premoistened and hermetically and antiseptically packed washcloths anymore. Washcloths to be used can be packed dry, which imposes less stringent requirements on hygiene and hence is cheaper. Another advantage of dry-packed washcloths is that they cannot dry out and hence have a longer storage life than prepacked, moist washcloths. This is also true of an opened package. Accordingly, after washing of a care recipient, the washcloths not used do not need to be discarded, but can be stored to be used subsequently.

Moreover, preservatives in the wash lotion can be much softer than the preservatives used in the prepacked moist wipes/washcloths according to the state of the art. It is even possible to make use of natural-based preservatives. Softer and/or natural preservatives are less aggressive and hence better for the skin. The chance of skin irritation is thereby reduced.

Another advantage of the wearable dispenser according to the invention over the above-described washing system with prepacked moist wipes/washcloths is that the use of packing material is reduced. In the washing system according to the state of the art, for each wash a package of moist wipes is opened and so this package ends up in the litterbin. The wash lotion in the wearable dispenser according to the invention can be in a package of a greater volume, providing an amount of wash lotion that is sufficient for carrying out a number of washings. With a 200 ml wash lotion holder, for example, about five washings can be carried out. The reduced consumption of packing material yields an environmental advantage again and, moreover, saves costs.

A further advantage of the wearable dispenser according to the invention is that per washing you always use precisely as many washcloths as you need for the washing. This in contrast to the use of moist wipes or washcloths which have been prepacked moist in a package intended for a single washing. With the washing system according to the state of the art, it happens regularly that the washing is already finished while the number of wipes in the package has not been used up completely. So, this leads to waste. The wearable dispenser of the invention always allows the user to "deliver to need". This is advantageous both from the environmental viewpoint and from the viewpoint of costs.

The invention further provides an article of clothing such as a pair of trousers, vest, or overall coat, which comprises the wearable dispenser according to the invention. The effects and advantages of the article of clothing are the same as the effects and the advantages of the wearable dispenser.

The invention provides in addition a method for washing a number of care recipients according to claim 24. More particularly, the invention provides a method which comprises providing a wearable dispenser according to the invention, attaching the wearable dispenser to a caregiver, providing a number of dry washcloths to the caregiver for the purpose of washing the number of care recipients, moistening at least one side of a dry washcloth of the number of dry washcloths for the purpose of washing a care recipient, and washing the care recipient with the moistened side of the washcloth.

The effects and advantages of the method are the same as the effects and the advantages of the wearable dispenser and the article of clothing.

It is noted that a wearable dispenser for dispensing liquid for the purpose of washing one's own hands is known from US2013/0099929. This publication is considered to be the closest prior art for the dispenser of claim 1 and the features mentioned in the precharacterizing portion of claim 1 are known from this publication. The wearable dispenser is represented in Figures 12-14 of this prior art publication. The known dispenser includes a liquid reservoir, a hand-powered, mechanical pump mechanism, and a single liquid dispensing opening. The aim of the known dispenser is to provide a solution to the problem of properly monitoring whether the user complies with the rules about washing one's own hands. To that end, the known dispenser is coupled with a sound generator, and the building in which the dispenser is used is provided with a sound sensing system which includes a communications system which is configured to transmit data representative of the sounds sensed by the sound sensing system. Based on the transmitted data, it can be determined per user whether such user has washed the hands sufficiently regularly in accordance with the rules.

Also US7988020B2 describes a wearable dispenser for dispensing liquid for washing the hands. In an embodiment shown therein, the dispenser includes a fluid reservoir which is compressible and whereby, through compression of the fluid reservoir, fluid can be dispensed via a single liquid dispensing opening into the palm by way of the increased pressure in the fluid reservoir. In another embodiment shown therein, the dispenser includes a hand-powered, mechanical pump mechanism for dispensing fluid via a single fluid dispensing opening.

With the provision of moist washcloths and the associated above-described issues, therefore, this publication does not have any connection.

US2004068824 describes an apparatus for washing persons. To this end, a complicated device which includes a mobile unit with a cleansing liquid reservoir and a rinsing liquid reservoir and pumps associated therewith. Further, the mobile unit includes a low-pressure source. The unit is connected via a hose to an applicator with which the cleansing liquid and/or the rinsing liquid can be dispensed to a body part to be cleaned of a care recipient. The low-pressure source is also connected via the hose to the applicator, so that dispensed cleansing liquid and/or rinsing liquid can be directly extracted again for drying the body part concerned. The publication concerned has no bearing on the problem to which the present invention provides a solution, namely providing moist washcloths in an efficient manner at the location of a care recipient.

Further elaborations of the invention are described in the subclaims and will be further clarified hereinafter on the basis of examples, with reference to the figures.

### BRIEF DESCRIPTION OF THE FIGURES

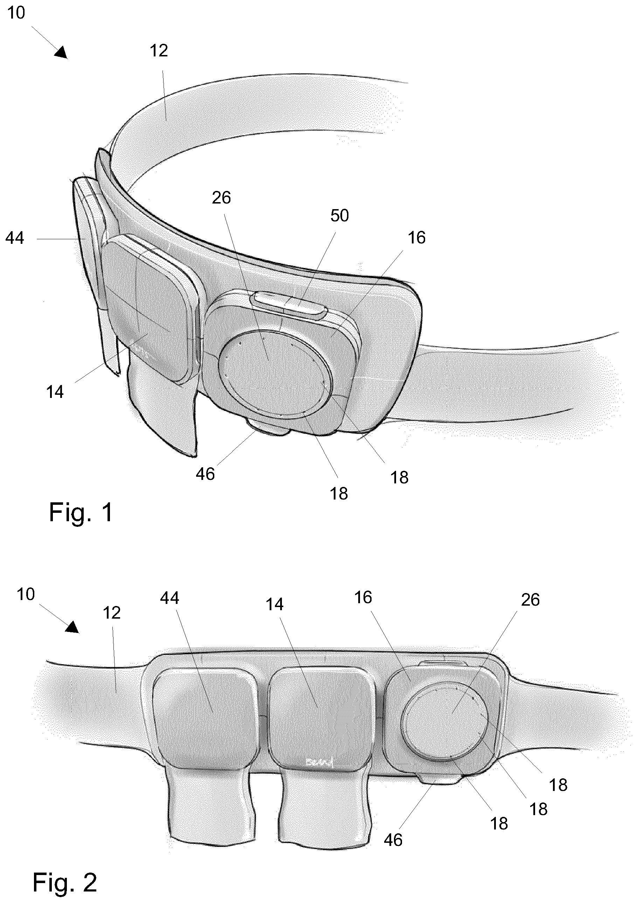

Fig. 1 shows a perspective view of an example of a wearable dispenser according to the invention;
Fig. 2 shows a front view of the example of Fig. 1;

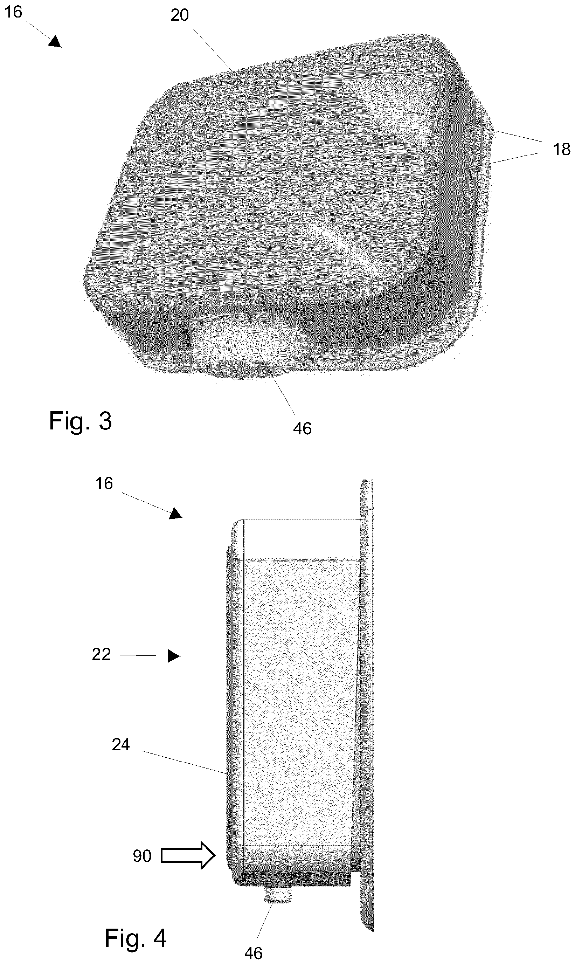

Fig. 3 shows a perspective front/bottom view of an example of a dispenser head according to the invention;
Fig. 4 shows a side view of an example of the dispenser head;
Fig. 5 shows an example of the inner side of a distribution disk and a wash lotion supply channel according to the invention;
Fig. 6 shows an example of a pattern of openings in a distribution disk according to the invention;
Fig. 7 shows an exemplary perspective view of a wash lotion holder according to the invention in an opened position;
Fig. 8 shows an example of the wearable dispenser provided with a washcloth holder;
Fig. 9 shows a schematic overview of an example of the transport mechanism, the liquid streams and the control according to the invention;
Fig. 10 shows an example of a schematic overview of a flow diagram for the control of the wearable dispenser; and
Fig. 11 shows an example of a user interface according to the invention.

### DETAILED DESCRIPTION

In the following detailed description, with the aid of reference numerals, reference is made to the examples that are represented in the figures. The embodiments that are described in the detailed description, however, are not limited to the examples that are shown in the figures but may also be implemented in a different manner than shown in the examples. The embodiments described in the detailed description should therefore be read and understood also without the reference numerals. The various embodiments to be described hereinafter can be applied in combination with each other or independently of each other.

Most generally, the invention provides a wearable dispenser 10 comprising a carrying support 12, a wash lotion holder 14, a dispenser head 16, and a transport mechanism 20. The wash lotion holder 14 is attached to the carrying support 12 and is configured for holding a wash lotion. The dispenser head 16 is attached to the carrying support 12 and is provided with multiple openings 18 configured for dispensing a dose of the wash lotion to a caregiver. The transport mechanism 20 is configured for transporting the dose of wash lotion from the wash lotion holder 14 to the dispenser head 16 to allow dispensing of the wash lotion to the caregiver.

The carrying support 12 can be, for example, a belt which is configured to be worn around a waist of the caregiver. But other implementations of the carrying support 12 also fall under the scope of protection of this description. For example, a rucksack, or a sling.

The carrying support 12 may further be provided with a holder 92 configured for holding dry washcloths. The caregiver then has the option of carrying along a supply of dry washcloths with the carrying support 12 of the wearable dispenser 10. In this way, during a wash round, the caregiver can grab a new washcloth every time to wash a new care recipient with it.

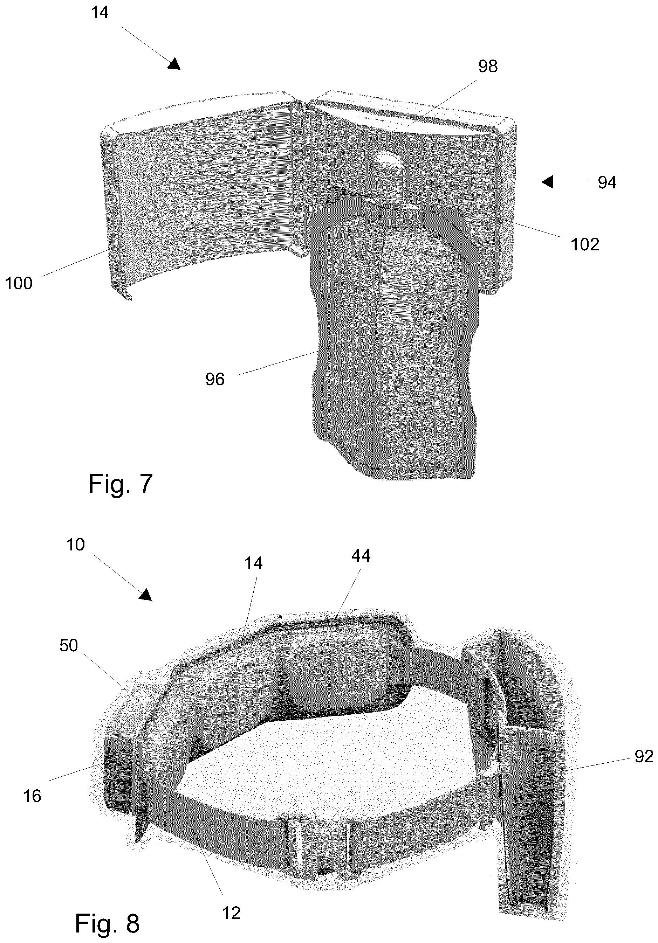

The wash lotion holder 14 may be implemented as one integral part. However, the wash lotion holder 14 may also be provided with a housing 94 and a replaceable refill pack viz. doy pouch 96 provided with the wash lotion. An example of such an implementation is represented in Figure 7. The housing 94 may consist of a body part 98 and a cover 100 hinging relative thereto. The doy pouch 96 can then be clamped in the housing 94 between the body part 98 and the cover 100. The doy pouch 96 may be provided with an outlet 102 which, if the doy pouch 96 is clamped in the housing 94, is in communication with the transport mechanism 20 to allow dispensing of the wash lotion to the caregiver.

By using a replaceable doy pouch 96, the doy pouch 96, if it is empty, can be simply removed from the housing 94, after which the wash lotion holder 14 can be provided with a new doy pouch 96.

In operation, the dose of wash lotion is dispensed through the multiple openings 18. The dispenser head 16 is provided with a front cap 22 which is movable in a direction substantially perpendicular to a front surface 24 of the front cap 22 for the purpose of providing an activation act 90 for activating the transport mechanism 20 for dispensing the dose of wash lotion via the multiple openings 18.

The multiple openings 18 may be distributed and in this way cover a particular surface area in which the dose of wash lotion is dispensed. In normal use of the wearable dispenser 10 it is not desirable that the dispenser head 16 dispenses wash lotion continuously. Ideally, the wearable dispenser 10 only dispenses a dose of wash lotion when the caregiver considers this necessary. To make this possible, the wearable dispenser 10 in this embodiment is provided with a front cap 22 which can provide an activation act 90. This activation act 90 is provided by pressing in the front cap 22. The activation act 90 activates the transport mechanism 20 so that the transport mechanism 20 dispenses a dose of wash lotion via the openings 18. Without the activation act 90, the transport mechanism 20 will not dispense a dose of wash lotion. What is achieved in this way is that a dose of wash lotion is dispensed only when the caregiver considers this necessary. The caregiver indicates this by pressing on the front cap 22 and thereby performing the activation act 90.

The openings 18 are provided in the front surface 24 of the front cap 22, such that pressing in with a washcloth on the front cap 22 directly leads to moistening of the washcloth. The spot which is to be pressed on to perform the activation act is then the same spot where the openings 18 are and where, therefore, the wash lotion is dispensed. Moistening of a washcloth *and* activation of the transport mechanism 20 are thereby made into a single act. The caregiver is thus enabled to use the wearable dispenser 10 more effectively and consequently can do his/her work faster.

The further effects and advantages of the wearable dispenser 10 have already been described in the summary, and these effects and advantages are understood to be inserted here by reference.

In an embodiment, the openings 18 may be provided in an area of the front cap 22 that is such that upon pressing in of the front cap 22 with the washcloth, a surface area of at least 15 cm² is moistened. To enable effective washing, the washcloth surface area moistened by the wearable dispenser 10 must be sufficient to allow a care recipient to be washed. An average surface area of a human palm is a suitable format for this.

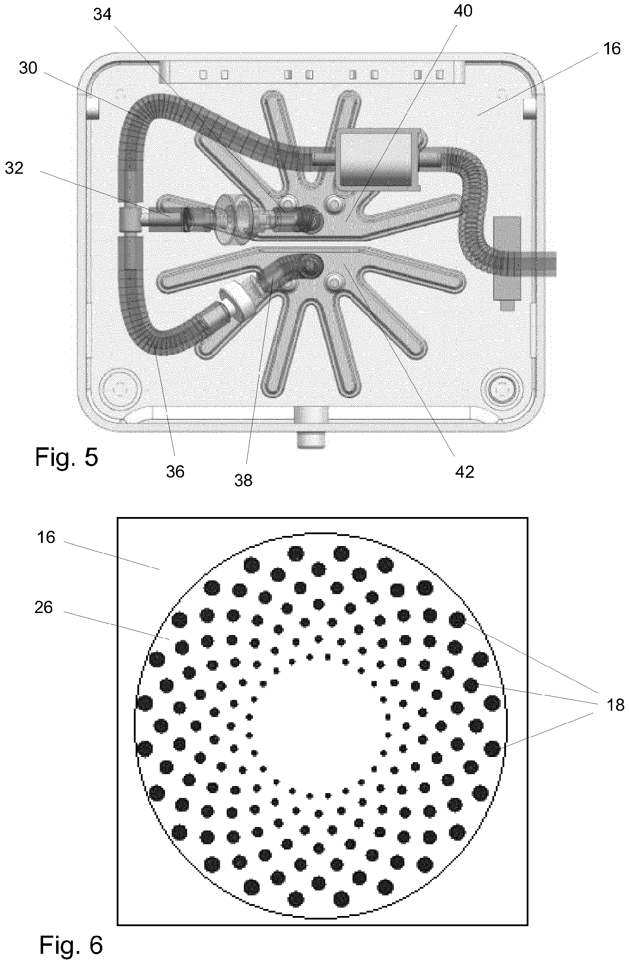

In an embodiment, the dispenser head 16 comprises a dispenser disk 26. An example of such a dispenser disk 26 is represented in Figure 6. By distributing the multiple openings 18 over the dispenser disk 26, it is possible by means of the dispenser disk 26 to distribute the dose of wash lotion evenly over the multiple openings 18. The multiple openings 18 are arranged at least along an edge of the dispenser disk 26. But the openings 18 may also be provided centrally in the dispenser disk 26. It is not necessary for each opening 18 to have a same size. The size of an opening 18 may differ according to the position of the respective opening 18 in the dispenser disk 26. The dispenser disk 26 may thus be provided with a pattern of openings 18 through which the dose of wash lotion is dispensed. This pattern may be so configured as to promote an optimum dispensing of the dose of wash lotion.

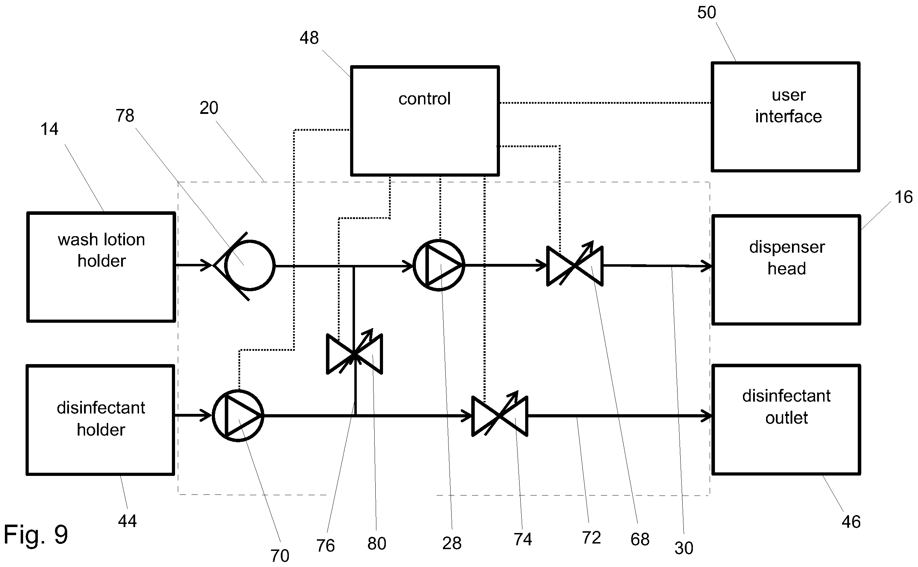

In an embodiment, the transport mechanism 20 is provided with a first pump 28. A schematic overview of an example of the transport mechanism 20 is represented in Figure 9. The first pump 28 may be arranged in or near the dispenser head 16, but also in or near the wash lotion holder 14. An inlet of the first pump 28 is connected with the wash lotion holder 14. The transport mechanism 20 is further provided with a wash lotion supply channel 30. The wash lotion supply channel 30 is connected at one end with the dispenser head 16, and the wash lotion supply channel 30 is connected at another end with an outlet of the first pump 28. The first pump 28 is configured for transporting the dose of wash lotion from the wash lotion holder 14 to the dispenser head 16. For the first pump 28, for example a Skoocom SC 1730PW diaphragm pump can be used. The advantage of a diaphragm pump is that the wash lotion cannot come into contact with a bearing of the diaphragm pump. The first pump may therefore be implemented as an electrically powered pump. Obviously, the wearable dispenser 10 is then provided with an electric source, such as a battery or a rechargeable storage battery. But other pumps, of course, are not excluded. For instance, the pump may also be of mechanical design and the mechanical energization of the pump may for instance be effected by pressing in the front cap 22. With the pressing in of the front cap 22, the mechanical pump is then directly energized and wash lotion dispensed.

The wash lotion supply channel 30 may be provided with a wash lotion control valve 68 downstream of the first pump 28 and upstream of the dispenser head 16. With such a control valve 68, the wash lotion supply channel 30 can be closed off by the control valve 68. This prevents leaking of the wash lotion out of the first pump 28 to the dispenser head 16. Also, with a wash lotion control valve 68, it is possible with the aid of the first pump 28 to build up pressure before the wash lotion control valve 68 is opened and the dose of wash lotion is transported by the first pump 28 to the dispenser head 16. In this way, sufficient pressure can be built up to overcome the resistance of the openings 18 in the dispenser head 16. This makes the dosing of the wash lotion better controllable.

The wash lotion supply channel 30 may, at the end connected with the dispenser head 16, split up into at least a first branch 32 and a second branch 36. An example of this is represented in Figure 5. The first branch 32 may be provided with a first check valve 34 and the second branch 36 may be provided with a second check valve 38. The first branch 32 may be connected with a first part 40 of the dispenser head 16 that comprises at least one first opening 18 of the multiple openings 18. The second branch 36 may be connected with a second part 42 of the dispenser head 16 that comprises at least one second opening 18 of the multiple openings 18. The first part 40 may, in use, be situated higher with respect to the horizontal than the second part 42. In an alternative embodiment, also three or more branches may be involved.

After the dose of wash lotion has been dispensed by the transport mechanism 20 via the dispenser head 16, a small remainder of wash lotion remains behind in the wash lotion supply channel 30 and the dispenser head 16. An effect that may thereupon occur is that this remainder is going to leak or drip from the dispenser head 16. By splitting the dose of wash lotion to be dispensed into two parts in the dispenser head and providing both parts of the wash lotion supply channel 30 with a check valve 34, 38, the remainder of the part of the dose of wash lotion that has gone to the first part 40 cannot leak via the two branches 32, 36 of the supply channel 30 to the second part 42. This counteracts the so-called after-drip of the dispenser head and will generally suffice to prevent such after-drip. The first check valve 34 and/or the second check valve 38 can be, for example, a duckbill valve.

In an embodiment, the wearable dispenser 10 further comprises a disinfectant holder 44 and a disinfectant outlet 46. The disinfectant holder 44 is attached to the carrying support 12 and is configured for holding a disinfectant. The disinfectant outlet 46 is configured for dispensing a dose of the disinfectant to the caregiver. The transport mechanism 20 is configured for transporting the dose of disinfectant from the disinfectant holder 44 to the disinfectant outlet 46 to allow dispensing of the disinfectant to the caregiver.

During or after washing a care recipient, the caregiver provided with the wearable dispenser 10 will also need to or want to carry out other actions than just washing alone. For example, the care recipient needs to be turned over, or something needs to be moved or handed to him. All acts whereby something is touched by the caregiver potentially entail a danger of contagion. The caregiver's hands may be infected with germs by touching a care recipient or any unsterile object. In order to counteract spread of such germs it is important, before a next care recipient is washed, to disinfect the caregiver's hands. Especially in a clinical environment, proper hand hygiene is of the essence for counteracting infections. Due to the wearable dispenser 10 in the present embodiment also comprising a disinfectant outlet 46 so that disinfectant can be dispensed, the caregiver does not need to walk to a tap or disinfection spot but can disinfect his/her hands directly with the disinfectant dispensed by the wearable dispenser 10. Thus, cross-infection between patients is hindered. This feature contributes towards enabling the caregiver to go from bed to bed very efficiently if he/she wants to wash multiple care recipients one after the other.

The dispenser head 16 may also comprise the disinfectant outlet 46. Examples of this are shown in the figures. In consequence, the caregiver can obtain both the wash lotion and the disinfectant from the dispenser head 16. The acts that are necessary for this can then be more or less the same, which promotes automation of these acts and increases the efficiency of the acts.

In addition to the use for the purpose of the caregiver, the disinfectant can also be used for other purposes. The transport mechanism 20 may be configured, for example, for transporting a rinse dose of the disinfectant from the disinfectant holder 44 to the at least one opening 18 of the dispenser head 16. In this way rinsing with the rinse dose of disinfectant disinfects the dispenser head 16. As the wash lotion is not disinfectant, germs contained in a remainder of wash lotion which remains behind in the dispenser head 16 after dispensing of a dose of wash lotion, may multiply. When the wearable dispenser 10 has not been used for some time, for example overnight, the next dose of wash lotion will contain an increased number of germs and thus signify an increased risk of infections of the care recipient. This can be prevented in that the transport mechanism 20 can be used to disinfect the dispenser head 16. This can preferably be done at set times, for example every evening when the wearable dispenser 10 is not going to be used anymore until the next morning.

In an embodiment, the wearable dispenser 10 comprises at least the following operating modes 82, 84, 86, 88: a wash mode 82, and a disinfect mode 84. In the wash mode 82, the transport mechanism 20 transports the dose of wash lotion to the dispenser head 16 and the dispenser head 16 dispenses the dose of wash lotion to the caregiver. In the disinfect mode 84, the transport mechanism 20 transports the dose of disinfectant to the disinfectant outlet 46 and the disinfectant outlet 46 dispenses the dose of disinfectant to the caregiver. The wearable dispenser 10 may further comprise the following operating modes 82, 84, 86, 88: a rinse mode 86 and an idle operating mode 88. In the rinse mode 86, the transport mechanism 20 transports the rinse dose of disinfectant from the disinfectant holder 44 to the dispenser head 16. In the idle operating mode 88, the transport mechanism 20 is in none of the above-mentioned operating modes 82, 84, 86, 88.

In an embodiment, the wearable dispenser 10 comprises a control 48. A schematic representation of an example of the control 48 is represented in Figure 9. The control 48 may be implemented as dedicated hardware. The control may also be implemented as an IC chip provided with software, which jointly take care of the proper operation. The control 48 is configured for operating the transport mechanism 20. The control 48 then arranges for the transport mechanism 20 to proceed to transport the wash lotion or the disinfectant. The control 48 may be configured to set the wearable dispenser 10 in one of the operating modes 82, 84, 86, 88. Such as the above-mentioned wash mode 82, the disinfect mode 84, the rinse mode 86, or the idle operating mode 88. However, the control may also be equipped to operate the wearable dispenser 10 in a different way.

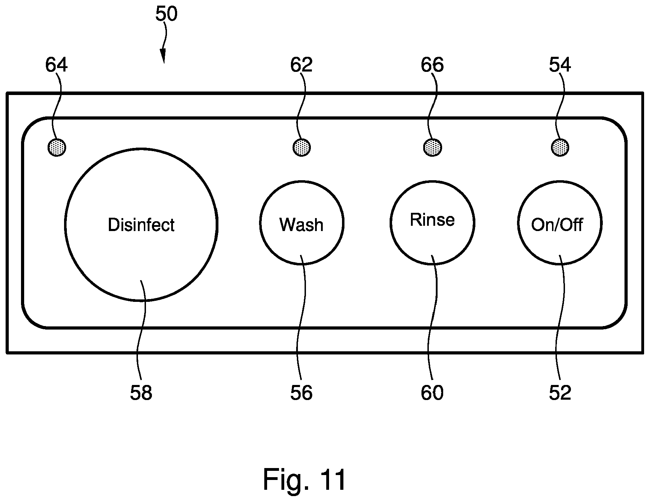

The wearable dispenser may further be provided with a user interface 50 which is configured to pass on commands from the caregiver to the control 48 and to pass on signals about the operation of the wearable dispenser 10 from the control 48 to the caregiver. An example of the user interface 50 is represented in Figure 11. The user interface 50 may be provided with at least one operating button 52, 56, 58, 60 which is configured to pass on a command from the caregiver to the control. The user interface 50 may also be provided with at least one indicator 54, 62, 64, 66 which is configured to pass on a signal about the operation of the wearable dispenser 10 from the control 48 to the caregiver.

By means of this user interface 50, the caregiver is able to operate the transport mechanism 20 and thereby the wearable dispenser 10. Instead of the operating button 52, 56, 58, 60 and the indicator 54, 62, 64, 66, of course, other interfaces are also possible. Such as, for example, a touchscreen or a screen with control panel.

The at least one operating button 52, 56, 58, 60 can comprise an on/off button 52 which is configured for turning the control 48 on or off. The at least one operating button 52, 56, 58, 60 can comprise in addition, or instead, a wash button 56 which is configured to give to the control 48 a command to set the wearable dispenser 10 in the wash mode 82. The at least one operating button 52, 56, 58, 60 can comprise in addition a disinfect button 58 which is configured to give to the control 48 a command to set the wearable dispenser 10 in the disinfect mode 84. The at least one operating button 52, 56, 58, 60 can additionally also comprise a rinse button 60 which is configured to give to the control 48 a command to set the wearable dispenser 10 in the rinse mode 86.

By the use of the at least one operating button 52, 56, 58, 60, the caregiver can pass on the desired operation of the wearable dispenser 10 to the transport mechanism 20 and, doing so, operate the wearable dispenser 10. An above-mentioned operating button 52, 56, 58, 60 may be implemented as a push button or as a touch button, or as any other operating button known in the field.

The at least one indicator 54, 62, 64, 66 can comprise an on-off indicator 54 which is configured to indicate to the caregiver whether the control 48 is on or off. The at least one indicator 54, 62, 64, 66 can also comprise a wash indicator 62, a disinfect indicator 64, and if applicable, a rinse indicator 66, which is configured to indicate whether the wearable dispenser 10 is in the corresponding operating mode 82, 84, 86, 88. An above-mentioned indicator 54, 62, 64, 66 can comprise an indication light. By placing the indication light, for example, next to an operating button 52, 56, 58, 60, this light can indicate, for instance by burning, that the transport mechanism 20 is in the operating mode 82, 84, 86, 88 which is associated with the respective operating button 52, 56, 58, 60.

In an embodiment, the control 48 may be configured for generating a signal via the at least one indicator 54, 62, 64, 66 when the wearable dispenser 10 must traverse a rinse procedure, for example upon expiry of a particular period of time after traversing a previous rinse procedure. In this way, it is actively brought to the caregiver's attention that rinsing/disinfecting of the wearable dispenser 10 is desired. This can be done in the manner described above, namely by, with the aid of one of the operating buttons 60, bringing the wearable dispenser 10 in the rinse mode and then performing the activation act 90, which can for instance consist in pressing in the front cap 22. The transport mechanism 20 then transports a rinse dose of the disinfectant from the disinfectant holder 44 to the at least one opening 18 of the dispenser head 16 to thus disinfect the dispenser head 16.

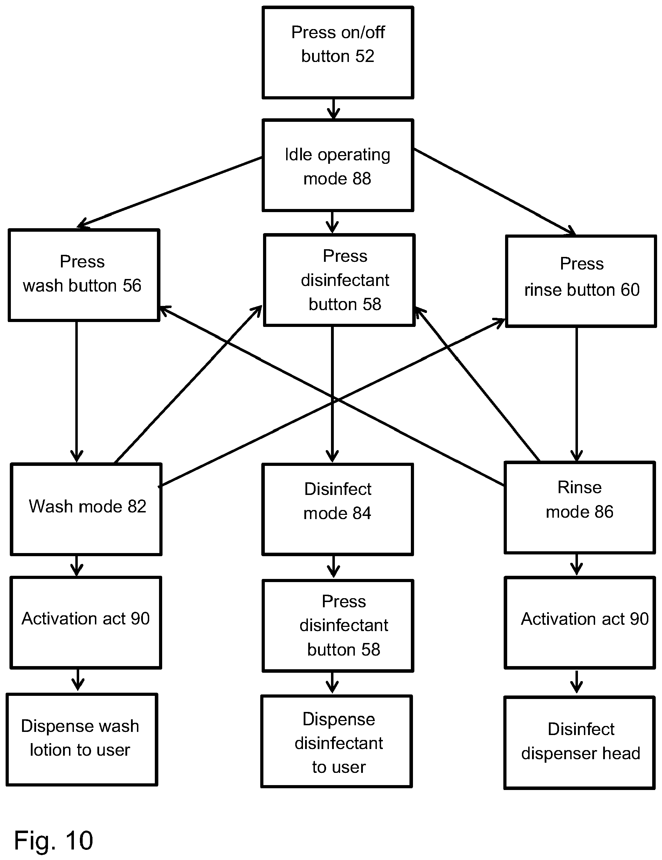

In an embodiment, the control 48 is configured to wait for an activation act 90 of the caregiver after the wearable dispenser 10 has been set in an operating mode 82, 84, 86, 88. The transport mechanism 20 carries out the acts associated with the above-mentioned operating mode 82, 84, 86, 88 after the activation act 90 has been performed by the caregiver. A schematic example of this setup is represented in Figure 10.

By making use of the activation act 90, the actions associated with an operating mode 82, 84, 86, 88 are not carried out directly after the wearable dispenser 10 has been set in this operating mode 82, 84, 86, 88. When the wearable dispenser 10 is set, for example, in the wash mode, the transport mechanism 20 does not directly start to dispense the dose of wash lotion to the caregiver. The transport mechanism 20 does not start to do so until after the activation act 90 has been performed by the caregiver. This has as an advantage that the caregiver can select and set the operating mode 82, 84, 86, 88 with one hand and can then position the same hand with respect to the dispenser head 16 or the disinfectant outlet 46 in a manner suitable for him to be able to receive the dispensed dose of wash lotion or dose of disinfectant. Also, the activation act 90 prevents an unwanted dispensing of wash lotion and/or disinfectant due to unintentional setting of the wearable dispenser 10 in an operating mode 82, 84, 86, 88, for example by pressing an operating button 52, 56, 58, 60 associated with the operating mode 82, 84, 86, 88.

The activation act 90 can comprise a pressing in of a side of the dispenser head 16. The activation act can also comprise the pressing of the operating button 52, 56, 58, 60 associated with the operating mode 82, 84, 86, 88 a second time. The control 48 may be configured, for example, to set the wearable dispenser 10 in the wash mode 82 upon the pressing of the wash button 56 by the caregiver, while the activation act 90 for the wash mode 82 comprises the pressing in of a side of the dispenser head 16. The control 48 may instead or additionally be configured, for example, to set the wearable dispenser 10 in the disinfect mode 84 upon the pressing of the disinfect button 58 by the caregiver, while the activation act 90 for the disinfect mode 84 comprises the pressing of the disinfect button 58 by the caregiver a second time. The control 48 may further be configured to set the wearable dispenser 10 in the rinse mode 86 upon the pressing of the rinse button 60 by the caregiver, while the activation act 90 for the rinse mode 86 comprises the pressing in of a side of the dispenser head 16.

In an embodiment, the transport mechanism 20 is provided with a second pump 70. The second pump 70 may, just like the first pump 28, be arranged in or near the dispenser head 16, but also in or near the wash lotion holder 14. Preferably, the two pumps 28, 70 are placed together. Preferably, both pumps 28, 70 are arranged in the dispenser head 16. An inlet of the second pump 70 is connected with the disinfectant holder 44. The transport mechanism 20 is further provided with a disinfectant supply channel 72. The disinfectant supply channel 72 is connected at one end with the disinfectant outlet 46, and the disinfectant supply channel 72 is connected at another end with an outlet of the second pump 70. The second pump 70 is configured for transporting the dose of disinfectant from the disinfectant holder 44 to the disinfectant outlet 46. For the second pump 70, just as for the first pump 28, for example a Skoocom SC1730PW diaphragm pump can be used. The advantage of a diaphragm pump is that the disinfectant cannot come into contact with a bearing of the diaphragm pump. But here too, of course, other pumps are not excluded. For example, the second pump can also be a mechanical pump which is mechanically operated, for example by pushing a disinfect button or a disinfect handle.

The disinfectant supply channel 72 may be provided with a disinfectant control valve 74 downstream of the second pump 70 and upstream of the dispenser head 16. With such a control valve 74, the disinfectant supply channel 72 can be closed off by the control valve 74. This prevents leaking of the disinfectant from the second pump 70 to the disinfectant outlet 46. Also, with a disinfectant control valve 74 it is possible, with the aid of the second pump 70, to build up pressure before the disinfectant control valve 74 is opened and the dose of disinfectant is transported by the second pump 70 to the disinfectant outlet 46. In this way, sufficient pressure can be built up to overcome the resistance of the disinfectant outlet 46. This makes the dosing of the disinfectant better controllable.

The transport mechanism 20 may further comprise a rinse duct 76 which by a first end is connected with the outlet of the second pump 70 and which by another end is connected with the inlet of the first pump 28. The second pump 70 may then be configured for transporting the rinse dose of disinfectant from the disinfectant holder 44 to the first pump 28. The first pump 28 may then be configured for transporting the rinse dose of disinfectant to the dispenser head 16. In this way, the rinse dose of disinfectant is transported via successively the second pump 70, the rinse duct 76, the first pump 28 and the wash lotion supply channel 30 to the dispenser head 16. At the dispenser head 16, it will disinfect the openings 18 and the dispenser head 16.

The transport mechanism 20 may further be provided with a check valve 78 between the wash lotion holder 14 and the rinse duct 76, so that the rinse dose of disinfectant cannot reach the wash lotion holder 14. Accordingly, this prevents the disinfectant reaching and contaminating the wash lotion in the wash lotion holder 14.

The rinse duct 76 may be provided with a rinse control valve 80 downstream of the second pump 70 and upstream of the first pump 28. With such a rinse control valve 80, the rinse duct 76 can be closed off by the control valve 80. Instead of the rinse control valve 80, the disinfectant control valve 74 can comprise a three-way control valve. A first outlet of this three-way control valve is then connected with the wash lotion supply channel 30 and a second outlet is then connected with the rinse duct 76. With such a three-way control valve, the rinse duct 76 can also be closed off. In both cases, leaking of the disinfectant from the second pump 70 to the first pump 28 is prevented.

This embodiment of the invention, of course, may be combined with the embodiment which includes the control configured for operating the transport mechanism 20. The control can then be configured for controlling the first pump 28 and the second pump 70. The control 48 may further be configured for controlling the wash lotion control valve 68, the disinfectant control valve 74 and possibly the rinse control valve 80. The control 48 can thus control all parts of the transport mechanism 20. The present embodiment, of course, may in addition also be combined with the embodiment which has different operating modes 82, 84, 86, 88. The control may then be configured to set the wearable dispenser 10 in one of the operating modes 82, 84, 86, 88.

In an embodiment, the carrying support 12 comprises the transport mechanism 20. By placing the transport mechanism 20 in the carrying support 12, the space available is effectively dealt with. For there is no need then to leave over or make space for the transport mechanism 20 on the carrying support or in one of the other parts of the wearable dispenser 10. Especially the supply channels 30, 72 and the rinse duct 76 can be properly concealed in the carrying support 12.

As already indicated above, in an embodiment, the wearable dispenser 10 may be provided with a first mechanical pump 28 for the transport of the wash lotion and, when the wearable dispenser 10 further includes a disinfectant holder 44, optionally with a second mechanical pump 70 for the transport of the disinfectant. This first mechanical pump can for instance be part of the wash lotion holder 14 and, if present, the second mechanical pump can be part of the disinfectant holder 44. The wash lotion holder 14 could then further include the dispenser head 16, so that a wash lotion unit is obtained which comprises the wash lotion holder 14, the first mechanical pump 28 and the dispenser head 16. Also, if desired, a disinfectant unit may be provided which comprises the disinfectant holder 44, the second mechanical pump 70 and the disinfectant outlet 46. It is possible to manufacture such an embodiment at a cost price so low that the wearable dispenser 10 can be implemented wholly or partly as a disposable. Thus, for example, the wash lotion unit and, if present, the disinfectant unit may be implemented as disposables.

In an embodiment, for example, the carrying support 12 can be reusable and the wash lotion unit can be replaced with a new specimen, again completely filled with wash lotion.

When the wearable dispenser 10 further includes a disinfectant unit, this unit too may be implemented as a disposable and be replaced with a new specimen, again completely filled with disinfectant.

Such embodiments provide the advantage that the cleaning of ducts is less critical.

In yet another alternative embodiment, even the carrying support, which may comprise, for example, a belt or an article of clothing, may be implemented as a disposable. In that case, therefore, the whole wearable dispenser 10 is implemented as a disposable unit.

The invention further provides an article of clothing, such as a pair of trousers, vest, or overall coat, which comprises the wearable dispenser 10 according to the invention. The effects and advantages of the article of clothing have already been described in the summary and these effects and advantages are understood to be inserted here by reference.

The invention provides in addition a method for washing a care recipient. The method comprises providing a wearable dispenser 10 according to the invention and attaching the wearable dispenser 10 to a caregiver. Further, the method comprises:
- providing a number of dry washcloths to the caregiver for the purpose of washing the number of caregivers;
- for the purpose of washing a care recipient, moistening at least one side of a dry washcloth of the number of dry washcloths; and
- washing the care recipient with the moistened side of the washcloth.

The effects and advantages of the method for washing a care recipient have already been described in the summary and these effects and advantages are understood to be inserted here by reference.

In a further elaboration of the method according to the invention, after the washing of a first care recipient of the number of care recipients, the caregiver can proceed directly to a second care recipient, the method then comprising:
- for the purpose of washing the second care recipient, moistening at least one side of a dry washcloth of the number of dry washcloths; and
- washing the second care recipient with the moistened side of the washcloth.

With the aid of this method, the caregiver can proceed from care recipient to care recipient without in the interim having to walk to another spot to wash his hands and/or having to warm up and bring along a pack of moist cloths. In this way, there is realized a considerably more efficient use of time of the caregiver who has to wash a ward of care recipients.

When, as described above, in an embodiment, the wearable dispenser is provided with a disinfectant holder 44 which is attached to the carrying support 12 and is provided with a disinfectant outlet 46 to allow dispensing of the disinfectant to the caregiver, the method may further comprise a step where between a washing of the first care recipient and a washing of the second care recipient the caregiver disinfects his hands with the aid of the disinfectant which is dispensed by the wearable dispenser. Thus the chance of cross-infection between patients is reduced still further, which is of utmost importance.

The invention is not limited to the examples shown in the figures. The above-described embodiments, as already indicated, may also be implemented differently than shown in the examples of the figures. The scope of protection is defined by the following claims in which the reference numerals have no limiting effect.

### Key to symbols

10 - wearable dispenser
12 - carrying support
14 - wash lotion holder
16 - dispenser head
18 - opening (in the dispenser head)
20 - transport mechanism
22 - front cap
24 - front surface
26 - dispenser disk
28 - first pump
30 - wash lotion supply channel
32 - first branch (of the wash lotion supply channel)
34 - first check valve (in the first branch of the ...)
36 - second branch (of the wash lotion supply channel)
38 - second check valve (in the second branch of the ...)
40 - first part (of the dispenser head)
42 - second part (of the dispenser head)
44 - disinfectant holder
46 - disinfectant outlet
48 - control
50 - user interface
52 - on/off button
54 - on-off indicator
56 - wash button
58 - disinfect button
60 - rinse button
62 - wash indicator
64 - disinfect indicator
66 - rinse indicator
68 - wash lotion control valve
70 - second pump
72 - disinfectant supply channel
74 - disinfectant control valve
76 - rinse duct
78 - check valve
80 - rinse control valve
82 - wash mode
84 - disinfect mode
86 - rinse mode
88 - idle operating mode
90 - activation act
92 - washcloth holder
94 - housing (of the wash lotion holder)
96 - doy pouch (of the wash lotion holder)
98 - body part (of the housing of the wash lotion holder)
100 - cover (of the housing of the wash lotion holder)
102 - outlet (of the doy pouch of the wash lotion holder)

## Claims

1. A wearable dispenser (10), comprising:
- a carrying support (12);
- a wash lotion holder (14) attached to the carrying support and configured for holding a wash lotion;
- a dispenser head (16) attached to the carrying support (12) and provided with at least one opening (18) configured for dispensing a dose of the wash lotion to a caregiver; and
- a transport mechanism (20) which is configured for transporting the dose of wash lotion from the wash lotion holder (14) to the dispenser head (16) to allow dispensing of the wash lotion to the caregiver,
**characterized in that** the at least one opening (18) of the dispenser head (16) comprises multiple openings (18) through which the dose of wash lotion, in operation, is dispensed, wherein the dispenser head (16) is provided with a front cap (22) which is movable in a direction substantially perpendicular to a front surface (24) of the front cap (22) for the purpose of providing an activation act (90) for activating the transport mechanism (20) for dispensing the dose of wash lotion via the openings (18), wherein the multiple openings (18) are provided in the front surface (24) of the front cap (22), such that pressing in with a washcloth on the front cap (22) leads directly to moistening of the washcloth.

2. The wearable dispenser according to claim 1, wherein the openings (18) are provided in an area of the front cap (22) that is such that upon the pressing in of the front cap (22) with the washcloth, at least 15 cm² is moistened.

3. The wearable dispenser according to claim 1 or 2, wherein the dispenser head (16) comprises a dispenser disk (26), and wherein the multiple openings (18) are arranged at least along an edge of the dispenser disk (26).

4. The wearable dispenser according to any one of the preceding claims, wherein the transport mechanism (20) is provided with a first pump (28) of which an inlet is connected with the wash lotion holder (14), wherein the transport mechanism (20) is further provided with a wash lotion supply channel (30) which at one end is connected with the dispenser head (16), and which at another end is connected with an outlet of the first pump (28), and wherein the first pump (28) is configured for transporting the dose of wash lotion from the wash lotion holder (14) to the dispenser head (16).

5. The wearable dispenser according to claim 4, wherein the wash lotion supply channel (30) at the end which is connected with the dispenser head (16) splits into at least a first branch (32) provided with a first check valve (34) and a second branch (36) provided with a second check valve (38), wherein the first branch (32) is connected with a first part (40) of the dispenser head (16) which comprises at least one first opening (18) of the multiple openings (18), and wherein the second branch (36) is connected with a second part (42) of the dispenser head (16) which comprises at least one second opening (18) of the multiple openings (18).

6. The wearable dispenser according to claim 5, wherein the first part (40), in use, is situated higher with respect to the horizontal than the second part (42).

7. The wearable dispenser according to claim 5 or 6, wherein the first check valve (34) and/or the second check valve (38) is a duckbill valve.

8. The wearable dispenser according to any one of the preceding claims, further comprising
- a disinfectant holder (44) attached to the carrying support (12) and configured for holding a disinfectant; and
- a disinfectant outlet (46) configured for dispensing a dose of the disinfectant to the caregiver,
wherein the transport mechanism (20) is configured for transporting the dose of disinfectant from the disinfectant holder (44) to the disinfectant outlet (46) to allow dispensing of the disinfectant to the caregiver.

9. The wearable dispenser according to claim 8, wherein the dispenser head (16) further comprises the disinfectant outlet (46).

10. The wearable dispenser according to claim 8 or 9, wherein the transport mechanism (20) is further configured for transporting a rinse dose of the disinfectant from the disinfectant holder (44) to the at least one opening (18) of the dispenser head (16) to allow rinse disinfecting of the dispenser head (16).

11. The wearable dispenser according to any one of claims 8-10, wherein the wearable dispenser (10) comprises at least the following operating modes (82, 84, 86, 88):
- a wash mode (82), wherein the transport mechanism (20) transports the dose of wash lotion to the dispenser head (16) and the dispenser head (16) dispenses the dose of wash lotion to the caregiver; and
- a disinfect mode (84), wherein the transport mechanism (20) transports the dose of disinfectant to the disinfectant outlet (46), and the disinfectant outlet (46) dispenses the dose of disinfectant to the caregiver.

12. The wearable dispenser according to claims 10 and 11, wherein the wearable dispenser (10) further comprises the following operating modes (82, 84, 86, 88):
- a rinse mode (86), wherein the transport mechanism (20) transports the rinse dose of disinfectant from the disinfectant holder (44) to the dispenser head (16); and
- an idle operating mode (88), wherein the transport mechanism (20) is in none of the aforementioned operating modes (82, 84, 86, 88).

13. The wearable dispenser according to any one of the preceding claims, further comprising a control (48) which is configured for operating the transport mechanism (20).

14. The wearable dispenser according to claim 11 or 12 and claim 13, wherein the control (48) is configured to set the wearable dispenser (10) in one of the operating modes (82, 84, 86, 88).

15. The wearable dispenser according to claim 13 or 14, further comprising a user interface (50) configured to pass on commands from the caregiver to the control (48) and to pass on signals about the operation of the wearable dispenser (10) from the control (48) to the caregiver, wherein the user interface (50) is provided with at least one operating button (52, 56, 58, 60) configured to pass on a command from the caregiver to the control and at least one indicator (54, 62, 64, 66) configured to pass on a signal about the operation of the wearable dispenser (10) from the control (48) to the caregiver.

16. The wearable dispenser according to claim 11 and claim 15, wherein the at least one operating button (52, 56, 58, 60) comprises a wash button (56) configured to give a command to the control (48) to set the wearable dispenser (10) in the wash mode (82), and a disinfect button (58) configured to give a command to the control (48) to set the wearable dispenser (10) in the disinfect mode (84).

17. The wearable dispenser according to claims 12 and 16, wherein the at least one operating button (52, 56, 58, 60) comprises a rinse button (60) configured to give a command to the control (48) to set the wearable dispenser (10) in the rinse mode (86).

18. The wearable dispenser according to any one of claims 15-17, in any case depending on claim 11 or 12, wherein the at least one indicator (54, 62, 64, 66) comprises a wash indicator (62), a disinfect indicator (64) and, if depending on claim 14, a rinse indicator (66), configured to indicate whether the wearable dispenser (10) is in the corresponding operating mode (82, 84, 86, 88).

19. The wearable dispenser according to any one of the preceding claims, in any case depending on claim 8, wherein the transport mechanism (20) is provided with a second pump (70) of which an inlet is connected with the disinfectant holder (44), wherein the transport mechanism (20) is further provided with a disinfectant supply channel (72) which at one end is connected with the disinfectant outlet (46), and which at another end is connected with an outlet of the second pump (70) and wherein the second pump (70) is configured for transporting the dose of disinfectant from the disinfectant holder (44) to the disinfectant outlet (46).

20. The wearable dispenser according to claim 19, in any case depending on claim 10, wherein the transport mechanism (20) is provided with a rinse duct (76) which by a first end is connected with the outlet of the second pump (70) and which by another end is connected with the inlet of the first pump (28), wherein the second pump (70) is configured for transporting the rinse dose of disinfectant from the disinfectant holder (44) to the first pump (28), and wherein the first pump (28) is configured for transporting the rinse dose of disinfectant to the dispenser head (16), and wherein the transport mechanism (20) is further provided with a check valve (78) between the wash lotion holder (14) and the rinse duct (76), so that the rinse dose of disinfectant cannot reach the wash lotion holder (14).

21. The wearable dispenser according to any one of the preceding claims, wherein the carrying support (12) comprises a belt which is configured to be worn around a waist of the caregiver.

22. The wearable dispenser according to any one of the preceding claims, wherein the carrying support (12) is further provided with a holder (92) configured for holding dry washcloths.

23. An article of clothing, such as a pair of trousers, vest, or overall coat, which comprises the wearable dispenser (10) according to any one of the preceding claims.

24. A method for washing a number of care recipients, comprising:
- providing a wearable dispenser (10) according to any one of claims 1-22 and attaching the wearable dispenser (10) to a caregiver;
- providing a number of dry washcloths to the caregiver for the purpose of washing the number of care recipients;
- for the purpose of washing a care recipient, moistening at least one side of a dry washcloth of the number of dry washcloths; and
- washing the care recipient with the moistened side of the washcloth.

25. The method according to claim 24, wherein after the washing of a first care recipient of the number of care recipients, the caregiver proceeds directly to a second care recipient, the method then comprising:
- for the purpose of washing the second care recipient, moistening at least one side of a dry washcloth of the number of dry washcloths; and
- washing the second care recipient with the moistened side of the washcloth.

26. The method according to claim 25, wherein the wearable dispenser in any case comprises the features of claim 8, wherein between a washing of the first care recipient and a washing of the second care recipient the caregiver disinfects his hands with the aid of the disinfectant which is dispensed by the wearable dispenser.

## Patentansprüche

1. Tragbarer Spender (10), umfassend:
- eine Tragestütze (12);
- einen Waschlotionshalter (14), der an der Tragestütze befestigt und zum Halten einer Waschlotion eingerichtet ist;
- einen Spenderkopf (16), der an der Tragestütze (12) befestigt und mit wenigstens einer Öffnung (18) versehen ist, die so eingerichtet ist, dass sie eine Dosis der Waschlotion an eine Pflegekraft abgibt; und
- einen Transportmechanismus (20), der so eingerichtet ist, dass er die Dosis der Waschlotion vom Waschlotionshalter (14) zum Spenderkopf (16) transportiert, um die Abgabe der Waschlotion an die Pflegekraft zu ermöglichen,
**dadurch gekennzeichnet, dass** die wenigstens eine Öffnung (18) des Spenderkopfs (16) mehrere Öffnungen (18) umfasst, durch die die Dosis von Waschlotion im Betrieb abgegeben wird, wobei der Spenderkopf (16) mit einer vorderen Kappe (22) versehen ist, die in einer Richtung im Wesentlichen senkrecht zu einer vorderen Fläche (24) der vorderen Kappe (22) beweglich ist, um einen Aktivierungsakt (90) zur Aktivierung des Transportmechanismus (20) zum Dosieren der Waschlotion über die Öffnungen (18) zur Verfügung zu stellen, wobei die mehreren Öffnungen (18) in der vorderen Fläche (24) der vorderen Kappe (22) vorgesehen sind, sodass ein Einpressen mit einem Waschlappen an der vorderen Kappe (22) direkt zu einer Befeuchtung des Waschlappens führt.

2. Tragbarer Spender nach Anspruch 1, wobei die Öffnungen (18) in einem Bereich der vorderen Kappe (22) vorgesehen sind, der derart ist, dass beim Einpressen der vorderen Kappe (22) mit dem Waschlappen mindestens 15 cm² befeuchtet werden.

3. Tragbarer Spender nach Anspruch 1 oder 2, wobei der Spenderkopf (16) eine Spenderscheibe (26) aufweist und wobei die mehreren Öffnungen (18) wenigstens entlang einer Kante der Spenderscheibe (26) angeordnet sind.

4. Tragbarer Spender nach einem der vorhergehenden Ansprüche, wobei der Transportmechanismus (20) mit einer ersten Pumpe (28) versehen ist, von der ein Einlass mit dem Waschlotionshalter (14) verbunden ist, wobei der Transportmechanismus (20) ferner mit einem Waschlotionsversorgungskanal (30) versehen ist, der an einem Ende mit dem Spenderkopf (16) verbunden ist und der an einem anderen Ende mit einem Auslass der ersten Pumpe (28) verbunden ist, und wobei die erste Pumpe (28) so eingerichtet ist, dass die Dosis der Waschlotion vom Waschlotionshalter (14) zum Spenderkopf (16) transportiert wird.

5. Tragbarer Spender nach Anspruch 4, wobei der Waschlotionsversorgungskanal (30) an dem Ende dasmit dem Spenderkopf (16) verbunden ist, in wenigstens einen ersten Zweig (32) mit einem ersten Rückschlagventil (34) und einen zweiten Zweig (36) mit einem zweiten Rückschlagventil (38) unterteilt ist, wobei der erste Zweig (32) mit einem ersten Teil (40) des Spenderkopfs (16) verbunden ist, der wenigstens eine erste Öffnung (18) der mehreren Öffnungen (18) umfasst, und wobei der zweite Zweig (36) mit einem zweiten Teil (42) des Spenderkopfs (16) verbunden ist, der wenigstens eine zweite Öffnung (18) der mehreren Öffnungen (18) umfasst.

6. Tragbarer Spender nach Anspruch 5, wobei das erste Teil (40), das verwendet wird, in Bezug auf die Waagerechte höher liegt als das zweite Teil (42).

7. Tragbarer Spender nach Anspruch 5 oder 6, wobei das erste Rückschlagventil (34) und/oder das zweite Rückschlagventil (38) ein Schnabelventil ist.

8. Tragbarer Spender nach einem der vorhergehenden Ansprüche, ferner umfassend:
- einen Desinfektionsmittelhalter (44), der an der Tragestütze (12) befestigt und für das Halten eines Desinfektionsmittels eingerichtet ist; und
- einen Desinfektionsmittelauslass (46), der so eingerichtet ist, dass er eine Dosis des Desinfektionsmittels an die Pflegekraft abgibt,
wobei der Transportmechanismus (20) so eingerichtet ist, dass er die Desinfektionsmitteldosis vom Desinfektionsmittelhalter (44) zum Desinfektionsmittelauslass (46) transportiert, um die Abgabe des Desinfektionsmittels an die Pflegekraft zu ermöglichen.

9. Tragbarer Spender nach Anspruch 8, wobei der Spenderkopf (16) ferner den Desinfektionsmittelauslass (46) aufweist.

10. Tragbarer Spender nach Anspruch 8 oder 9, wobei der Transportmechanismus (20) ferner dazu eingerichtet ist, eine Spüldosis des Desinfektionsmittels von dem Desinfektionsmittelhalter (44) zu der wenigstens einen Öffnung (18) des Spenderkopfs (16) zu transportieren ist, um eine Spüldesinfektion des Spenderkopfs (16) zu ermöglichen.

11. Tragbarer Spender nach einem der Ansprüche 8 bis 10, wobei der tragbare Spender (10) wenigstens die folgenden Betriebsmodi (82, 84, 86, 88) aufweist:
- einen Waschmodus (82), wobei der Transportmechanismus (20) die Dosis der Waschlotion zum Spenderkopf (16) transportiert und der Spenderkopf (16) die Dosis der Waschlotion an die Pflegekraft ausgibt; und
- einen Desinfektionsmodus (84), wobei der Transportmechanismus (20) die Desinfektionsmitteldosis zum Desinfektionsmittelauslass (46) transportiert und der Desinfektionsmittelauslass (46) die Desinfektionsmitteldosis an die Pflegekraft abgibt.

12. Tragbarer Spender nach den Absprüchen 10 und 11, wobei der tragbare Spender (10) ferner die folgenden Betriebsmodi (82, 84, 86, 88) umfasst:
- einen Spülmodus (86), wobei der Transportmechanismus (20) die Spüldosis des Desinfektionsmittels vom Desinfektionsmittelhalter (44) zum Spenderkopf (16) transportiert; und
- einen Leerlaufbetriebsmodus (88), wobei sich der Transportmechanismus (20) in keiner der oben genannten Betriebsmodi (82, 84, 86, 88) befindet.

13. Tragbarer Spender nach einem der vorhergehenden Ansprüche, ferner umfassend eine Steuerung (48), die zum Bedienen des Transportmechanismus (20) eingerichtet ist.

14. Tragbarer Spender nach Anspruch 11 oder 12 und Anspruch 13, wobei die Steuerung (48) dazu eingerichtet ist, den tragbaren Spender (10) in einen der Betriebsmodi (82, 84, 86, 88) einzustellen.

15. Tragbarer Spender nach Anspruch 13 oder 14, ferner umfassend eine Benutzerschnittstelle (50), die so eingerichtet ist, dass sie Befehle von der Pflegekraft an die Steuerung (48) weitergibt und Signale über die Bedienung des tragbaren Spenders (10) von der Steuerung (48) an die Pflegekraft weitergibt, wobei die Benutzerschnittstelle (50) mit wenigstens einer Bedientaste (52, 56, 58, 60) ausgestattet ist, die so eingerichtet ist, dass sie ein Kommando von der Pflegekraft an die Steuerung und wenigstens einen Indikator (54, 62, 64, 66) weitergibt und so eingerichtet ist, dass sie ein Signal über den Betrieb des tragbaren Spenders (10) von der Steuerung (48) an die Pflegekraft weitergibt.

16. Tragbarer Spender nach Anspruch 11 und 15, wobei die wenigstens eine Bedientaste (52, 56, 58, 60) eine Waschtaste (56) umfasst, die so eingerichtet ist, dass sie der Steuerung (48) ein Kommando zum Einstellen des tragbaren Spenders (10) im Waschmodus (82) erteilt, und eine Desinfektionstaste (58), die so eingerichtet ist, dass sie der Steuerung (48) ein Kommando zum Einstellen des tragbaren Spenders (10) im Desinfektionsmodus (84) erteilt.

17. Tragbarer Spender nach den Ansprüchen 12 und 16, wobei der wenigstens eine Bedienknopf (52, 56, 58, 60) eine Spültaste (60) aufweist, die so eingerichtet ist, dass sie der Steuerung (48) einen Befehl gibt, den tragbaren Spender (10) in den Spülmodus (86) einzustellen.

18. Tragbarer Spender nach einem der Ansprüche 15 bis 17, jedenfalls abhängig von Anspruch 11 oder 12, wobei der wenigstens eine Indikator (54, 62, 64, 66) einen Waschindikator (62), einen Desinfektionsindikator (64) und, wenn je nach Anspruch 14, einen Spülindikator (66) aufweist, der so eingerichtet ist, dass er anzeigt, ob sich der tragbare Spender (10) in dem betreffenden Betriebsmodus (82, 84, 86, 88) befindet.

19. Tragbarer Spender nach einem der vorhergehenden Ansprüche, jedenfalls abhängig von Anspruch 8, wobei der Transportmechanismus (20) mit einer zweiten Pumpe (70) versehen ist, von der ein Einlass mit dem Desinfektionsmittelhalter (44) verbunden ist, wobei der Transportmechanismus (20) ferner mit einem Desinfektionsmittelversorgungskanal (72) versehen ist, der an einem Ende mit dem Desinfektionsmittelauslass (46) verbunden ist, und der an einem anderen Ende mit einem Auslass der zweiten Pumpe (70) verbunden ist und wobei die zweite Pumpe (70) zum Transport der Desinfektionsmitteldosis vom Desinfektionsmittelhalter (44) zum Desinfektionsmittelauslass (46) eingerichtet ist.

20. Tragbarer Spender nach Anspruch 19, jedenfalls abhängig von Anspruch 10, wobei der Transportmechanismus (20) mit einem Spülkanal (76) versehen ist, der durch ein erstes Ende mit dem Auslass der zweiten Pumpe (70) verbunden ist und der durch ein anderes Ende mit dem Einlass der ersten Pumpe (28) verbunden ist, wobei die zweite Pumpe (70) zum Transport der Spülmitteldosis von dem Desinfektionsmittelhalter (44) zur ersten Pumpe (28) eingerichtet ist, und wobei die erste Pumpe (28) zum Transportieren der Spüldosis von Desinfektionsmittel zum Spenderkopf (16) eingerichtet ist und wobei der Transportmechanismus (20) ferner mit einem Rückschlagventil (78) zwischen dem Waschlotionshalter (14) und dem Spülkanal (76) versehen ist, sodass die Spüldosis von Desinfektionsmittel den Waschlotionshalter (14) nicht erreichen kann.

21. Tragbarer Spender nach einem der vorhergehenden Ansprüche, wobei die Tragestütze (12) einen Gurt aufweist, der so eingerichtet ist, dass er um die Taille der Pflegekraft getragen werden kann.

22. Tragbarer Spender nach einem der vorhergehenden Ansprüche, wobei der Träger (12) ferner mit einem Halter (92) versehen ist, der zum Halten trockener Waschlappen eingerichtet ist.

23. Bekleidungsgegenstand, wie eine Hose, Weste oder ein Mantel, der den tragbaren Spender (10) gemäß einem der vorhergehenden Ansprüche umfasst.

24. Verfahren zum Waschen einer Reihe von Pflegeempfängern, umfassend:
- Bereitstellen eines tragbaren Spenders (10) nach einem der Ansprüche 1-22 und Anbringen des tragbaren Spenders (10) an eine Pflegekraft;
- Bereitstellung einer Reihe von trockener Waschlappen für die Pflegekraft zum Waschen der Anzahl der Pflegeempfänger;
- um einen Pflegeempfänger zu Waschen, Befeuchten wenigstens einer Seite eines trockenen Waschlappens aus der Anzahl der Trockenwaschlappen; und
- Waschen des Pflegeempfängers mit der angefeuchteten Seite des Waschlappens.

25. Verfahren nach Anspruch 24, wobei nach dem Waschen eines ersten Pflegeempfängers aus der Anzahl der Pflegeempfänger, die Pflegekraft direkt zu einem zweiten Pflegeempfänger übergeht, wobei das Verfahren dann umfasst:
- um den zweiten Pflegeempfänger zu waschen, Befeuchten wenigstens einer Seite eines trockenen Waschlappens aus der Anzahl der trockenen Waschlappen; und
- Waschen des zweiten Pflegeempfängers mit der angefeuchteten Seite des Waschlappens.

26. Verfahren nach Anspruch 25, wobei der tragbare Spender in jedem Fall die Merkmale des Anspruchs 8 aufweist, wobei zwischen einem Waschen des ersten Pflegeempfängers und einem Waschen des zweiten Pflegeempfängers die Pflegekraft ihre Hände mit Hilfe des Desinfektionsmittels desinfiziert, das vom tragbaren Spender abgegeben wird.

## Revendications

1. Distributeur à porter sur soi (10), comprenant :
un support porteur (12);
un support de lotion de lavage (14) fixé au support de transport et configuré pour contenir une lotion de lavage ;
une tête de distribution (16) fixée au support de portage (12) et pourvue d'au moins une ouverture (18) configurée pour distribuer une dose de la lotion de lavage à un soignant ; et
un mécanisme de transport (20) qui est configuré pour transporter la dose de lotion de lavage du support de lotion de lavage (14) à la tête de distribution (16) pour permettre la distribution de la lotion de lavage au soignant,
**caractérisé en ce que** le au moins un l'ouverture (18) de la tête de distribution (16) comprend plusieurs ouvertures (18) à travers lesquelles la dose de lotion de lavage, en fonctionnement, est distribuée, la tête de distribution (16) étant pourvue d'un capuchon avant (22) qui est mobile dans une direction sensiblement perpendiculaire à une surface avant (24) du capuchon avant (22) dans le but de fournir un acte d'activation (90) pour activer le mécanisme de transport (20) pour distribuer la dose de lotion de lavage via les ouvertures (18), dans lequel les multiples ouvertures (18) sont prévues dans la surface avant (24) du capuchon avant (22), de sorte qu'une pression avec un gant de toilette sur le capuchon avant (22) conduit directement à l'humidification du gant de toilette.

2. Distributeur à porter sur soi selon la revendication 1, dans lequel les ouvertures (18) sont prévues dans une zone du capuchon avant (22) qui est telle que lors de l'enfoncement du capuchon avant (22) avec le gant de toilette, au moins 15 cm² est humidifié.

3. Distributeur à porter sur soi selon la revendication 1 ou 2, dans lequel la tête de distribution (16) comprend un disque de distribution (26), et dans lequel les multiples ouvertures (18) sont disposées au moins le long d'un bord du disque de distribution (26).

4. Distributeur à porter sur soi selon l'une quelconque des revendications précédentes, dans lequel le mécanisme de transport (20) est pourvu d'une première pompe (28) dont une entrée est reliée au support de lotion de lavage (14), dans lequel le mécanisme de transport (20) est en outre pourvu d'un canal d'alimentation en lotion de lavage (30) qui, à une extrémité, est relié à la tête de distribution (16), et qui, à une autre extrémité, est relié à une sortie de la première pompe (28), et dans lequel la première pompe (28) est configurée pour transporter la dose de lotion de lavage du support de lotion de lavage (14) vers la tête de distribution (16).

5. Distributeur à porter sur soi selon la revendication 4, dans lequel le canal d'alimentation en lotion de lavage (30) à l'extrémité qui est reliée à la tête de distribution (16) se divise en au moins une première branche (32) munie d'un premier clapet anti-retour (34) et une deuxième branche (36) munie d'un deuxième clapet anti-retour (38), la première branche (32) étant reliée à une première partie (40) de la tête de distribution (16) qui comprend au moins une première ouverture (18) parmi les ouvertures multiples (18), et dans lequel la deuxième branche (36) est reliée à une deuxième partie (42) de la tête de distribution (16) qui comprend au moins une deuxième ouverture (18) parmi les ouvertures multiples (18) .

6. Distributeur à porter sur soi selon la revendication 5, dans lequel la première partie (40), en utilisation, est située plus haut par rapport à l'horizontale que la deuxième partie (42).

7. Distributeur à porter sur soi selon la revendication 5 ou 6, dans lequel le premier clapet anti-retour (34) et/ou le deuxième clapet anti-retour (38) est un clapet à bec de canard.

8. Distributeur à porter sur soi selon l'une quelconque des revendications précédentes, comprenant en outre
un support de désinfectant (44) fixé au support de transport (12) et configuré pour contenir un désinfectant ; et
une sortie de désinfectant (46) configurée pour distribuer une dose de désinfectant au soignant,
dans lequel le mécanisme de transport (20) est configuré pour transporter la dose de désinfectant du support de désinfectant (44) à la sortie de désinfectant (46) pour permettre la distribution du désinfectant au soignant.

9. Distributeur à porter sur soi selon la revendication 8, dans lequel la tête de distribution (16) comprend en outre la sortie de désinfectant (46).

10. Distributeur à porter sur soi selon la revendication 8 ou 9, dans lequel le mécanisme de transport (20) est en outre configuré pour transporter une dose de rinçage du désinfectant depuis le support de désinfectant (44) vers au moins une ouverture (18) de la tête de distribution (16) pour permettre une désinfection par rinçage de la tête de distribution (16).

11. Distributeur à porter sur soi selon l'une quelconque des revendications 8 à 10, dans lequel le distributeur à porter sur soi (10) comprend au moins les modes de fonctionnement suivants (82, 84, 86, 88) :
- un mode de lavage (82), dans lequel le mécanisme de transport (20) transporte la dose de lotion de lavage vers la tête de distribution (16) et la tête de distribution (16) distribue la dose de lotion de lavage au soignant ; et
- un mode désinfection (84), dans lequel le mécanisme de transport (20) transporte la dose de désinfectant vers la sortie de désinfectant (46), et la sortie de désinfectant (46) distribue la dose de désinfectant au soignant.

12. Distributeur à porter sur soi selon les revendications 10 et 11, dans lequel le distributeur à porter sur soi (10) comprend en outre les modes de fonctionnement suivants (82, 84, 86, 88) :
- un mode de rinçage (86), dans lequel le un mécanisme de transport (20) transporte la dose de rinçage de désinfectant du support de désinfectant (44) à la tête de distribution (16) ; et
- un mode de fonctionnement inactif (88), dans lequel le mécanisme de transport (20) n'est dans aucun des modes de fonctionnement susmentionnés (82, 84, 86, 88).

13. Distributeur à porter sur soi selon l'une quelconque des revendications précédentes, comprenant en outre une commande (48) qui est configurée pour actionner le mécanisme de transport (20).

14. Distributeur à porter sur soi selon la revendication 11 ou 12 et la revendication 13, dans lequel la commande (48) est configurée pour régler le distributeur à porter sur soi (10) dans l'un des modes de fonctionnement (82, 84, 86, 88).

15. Distributeur à porter sur soi selon la revendication 13 ou 14, comprenant en outre une interface utilisateur (50) configurée pour transmettre des commandes du soignant à la commande (48) et pour transmettre des signaux concernant le fonctionnement du distributeur à porter sur soi (10) depuis la commande (48) au soignant, dans lequel l'interface utilisateur (50) est pourvue d'au moins un bouton de commande (52, 56, 58, 60) configuré pour transmettre une commande du soignant à la commande et au moins un indicateur (54, 62, 64, 66) configuré pour transmettre un signal concernant le fonctionnement du distributeur à porter sur soi (10) de la commande (48) au soignant.

16. Distributeur à porter sur soi selon la revendication 11 et la revendication 15, dans lequel le au moins un bouton de fonctionnement (52, 56, 58, 60) comprend un bouton de lavage (56) configuré pour donner une commande à la commande (48) pour régler le distributeur à porter sur soi (10) en mode lavage (82), et un bouton de désinfection (58) configuré pour donner une commande à la commande (48) pour mettre le distributeur à porter sur soi (10) en mode désinfection (84).

17. Distributeur à porter sur soi selon les revendications 12 et 16, dans lequel le au moins un bouton de fonctionnement (52, 56, 58, 60) comprend un bouton de rinçage (60) configuré pour donner une commande à la commande (48) pour régler le distributeur à porter sur soi (10) en mode rinçage (86).

18. Distributeur à porter sur soi selon l'une quelconque des revendications 15 à 17, dans tous les cas selon la revendication 11 ou 12, dans lequel le au moins un indicateur (54, 62, 64, 66) comprend un indicateur de lavage (62), un indicateur de désinfection (64) et, si dépendant de la revendication 14, un indicateur de rinçage (66), configuré pour indiquer si le distributeur à porter sur soi (10) est dans le mode de fonctionnement correspondant (82, 84, 86, 88).

19. Distributeur à porter sur soi selon l'une quelconque des revendications précédentes, en tout cas dépendant de la revendication 8, dans lequel le mécanisme de transport (20) est muni d'une deuxième pompe (70) dont une entrée est reliée au support de désinfectant (44), dans lequel le mécanisme de transport (20) est en outre pourvu d'un canal d'alimentation en désinfectant (72) qui, à une extrémité, est relié à la sortie de désinfectant (46), et qui, à une autre extrémité, est relié à une sortie de la deuxième pompe (70) et dans lequel la deuxième pompe (70) est configurée pour transporter la dose de désinfectant depuis le support de désinfectant (44) à la sortie de désinfectant (46).

20. Distributeur à porter sur soi selon la revendication 19, en tout cas dépendant de la revendication 10, dans lequel le mécanisme de transport (20) est pourvu d'un conduit de rinçage (76) et qui par une première extrémité est relié à la sortie de la deuxième pompe (70) et qui par une autre extrémité est reliée à l'entrée de la première pompe (28), la deuxième pompe (70) étant configurée pour transporter la dose de rinçage de désinfectant du support de désinfectant (44) à la première pompe (28), et dans lequel la première pompe (28) est configurée pour transporter la dose de rinçage de désinfectant vers la tête de distribution (16), et dans lequel le mécanisme de transport (20) est en outre pourvu d'un clapet anti-retour (78) entre le support de lotion de lavage (14 ) et le conduit de rinçage (76), de sorte que la dose de rinçage de désinfectant ne puisse pas atteindre le support de lotion de lavage (14).

21. Distributeur à porter sur soi selon l'une quelconque des revendications précédentes, dans lequel le support de portage (12) comprend une ceinture qui est configurée pour être portée autour de la taille du soignant.

22. Distributeur à porter sur soi selon l'une quelconque des revendications précédentes, dans lequel le support de transport (12) est en outre pourvu d'un support (92) configuré pour maintenir des gants de lavage sèches.

23. Article vestimentaire, tel qu'un pantalon, un gilet ou une blouse, qui comprend le distributeur à porter sur soi (10) selon l'une quelconque des revendications précédentes.

24. Procédé pour laver un certain nombre de bénéficiaires de soins, comprenant :
- la fourniture d'un distributeur à porter sur soi (10) selon l'une quelconque des revendications 1 à 22 et la fixation du distributeur à porter sur soi (10) à un soignant ;
- la fourniture d'un certain nombre de gants de lavage sèches au soignant dans le but de laver le nombre de bénéficiaires de soins ;
- dans le but de laver un bénéficiaire des soins, la humidification d'au moins un côté d'une gant de lavage sèche parmi le nombre de gants de lavage sèches ; et
- laver le bénéficiaire des soins avec le côté humidifié de la gant de lavage.

25. Procédé selon la revendication 24, dans lequel après le lavage d'un premier bénéficiaire de soins du nombre de bénéficiaires de soins, le soignant procède directement à un deuxième bénéficiaire de soins, le procédé comprenant alors :
- dans le but de laver le deuxième bénéficiaire de soins, humidifier au moins une face d'un gant de toilette sec parmi le nombre de gants de toilette secs ; et
- laver le deuxième bénéficiaire de soins avec le côté humidifié du gant de lavage.

26. Procédé selon la revendication 25, dans lequel le distributeur à porter sur soi comprend dans tous les cas les caractéristiques de la revendication 8, dans lequel entre un lavage du premier bénéficiaire de soins et un lavage du deuxième bénéficiaire de soins, le soignant se désinfecte les mains à l'aide du désinfectant qui est distribué par le distributeur à porter sur soi.
